# EUROPEAN PATENT APPLICATION

(11) **EP 1 803 475 A2**
(43) Date of publication of application: **04.07.2007**
(21) Application number: 06256583.3
(22) Date of filing: 27.12.2006
(51) Int. Cl.: A61L 27/50

(54) **Method to develop an organised microstructure within an implantable medical device**

(30) Priority: 29.12.2005 US 321547
(71) Applicant: Cordis Corporation, Miami Lakes, FL 33014 (US)
(72) Inventor: Burgermeister, Robert, Bridgewater, NJ 08807 (US); Grishaber, Randy-David Bruce, Asbury, NJ 08802 (US); Park, Jin S., Parsippany, NJ 07054 (US)
(74) Representative: Belcher, Simon James

(57) **Abstract**

A method to facilitate the ability to engineer an organized microstructure within a monolithically homogeneous implantable biomaterial, particularly metallic alloys, is provided. By starting with an ultra-fine grained, equiaxed microstructure having an averaged linear grain size of less than 5 µm (5 microns), a functionally graded microstructure is developed by establishing a temperature gradient across the cross sectional dimension. In the case of a tubular product form, this dimension would be framed by the outer and inner surfaces. By ensuring one surface is held at a substantially lower temperature than the other surface a temperature gradient is developed. The present invention also provides an implantable medical device which includes an organized microstructure. In accordance with the present invention, the organized microstructure includes a first surface region having said ultra-fine grained, equiaxed microstructure and a second surface region comprising a recrystallized, coarser grained microstructure having an average linear grain size of greater than 25 µm (25 microns), wherein the first and second surface regions are separated by a transition region.

## Description

The present invention relates to implantable medical devices, and in particular to implantable medical devices which include an organized microstructure within the device itself. The present invention also relates to a method of fabricating such an organized microstructure within an implantable medical device.

Implantable medical devices such as, for example, stents, blood filters, artificial heart valves, vascular occluders, aneurismal coils, prosthetic grafts including stent-grafts and the like are typically subjected to hostile working conditions and environments when inserted into a patient. For example, stents and blood filters are introduced into a patient's body while in a compressed shape and are thereafter expanded to a final, useful shape when positioned to a target location within the body. After deployment, the implantable devices should have sufficient physical, biological and mechanical properties to perform throughout the expected useful lifetime of the medical device. In addition, other implantable medical devices may include bone pins, shunts, screws, spinal fixation devices, and implantable orthopedic prosthetic devices such as hips, knee joints and spinal discs.

In view of the stringent requirements of medical devices, the processes used to form these devices must be accurate and reproducible, and obtain the desired dimensional, compositional and mechanical properties. Conventional production processes, however, are often complex and expensive. Moreover, most of the manufacturing steps associated with conventional processes introduces defects into the metallic structure of the formed implantable medical device. The defects can include excessive oxidation, localized deformation, surface flaws and the like. These defects often reduce desired properties, such as strength, fatigue resistance and corrosion resistance.

The performance properties of the implantable medical device are not only affected by manufacturing processes, but are also affected by the material properties of the raw material. For instance, if the wire or tube used to form an implantable medical device contains material or structural defects, the formed medical device may also often contain similar or greater defects. Some defects in the formed device may be reduced by techniques, such as annealing, but these techniques often impart other undesirable effects. For instance, annealing often requires high temperature treatment (on the order of about 0.88 to 0.90 homologous temperature or greater, where the homologous temperature is the industrially accepted liquidus temperature also generally referred to as the melting temperature of the material) of a metallic device to recrystallize its microstructure to reduce grain size and residual stress. Such a high temperature treatment can often impart physical deformation of the device due to thermal heating and cooling steps or due to the change in the microstructure itself.

One problem with the conventional annealing process mentioned above is that a monolithic device is formed in which the microstructure of the material throughout the entire device is substantially the same. The formation of such monolithic devices having substantially the same microstructure oftentimes leads to crack nucleation and propagation during the use of the implantable medical device. Moreover, the prior art devices which are fabricated from a material having substantially the same microstructure are fatigue prone since the highest strength regions are not necessarily located where the highest levels of stress are expected.

Accordingly, a need exists for a process to form a medical device without the disadvantages of the prior art. Furthermore, a need exists for a medical device with improved biocompatability and mechanical properties.

The present invention provides a method to facilitate the ability to engineer an organized microstructure within a monolithically homogeneous implantable biocompatible material, particularly metallic alloys. By starting with an ultra-fine grained, equiaxed microstructure having an averaged linear grain size of less than 5 microns, a functionally graded microstructure is developed by establishing a temperature gradient across the cross sectional dimension. In the case of a tubular product form, this dimension would be framed by the outer and inner surfaces (i.e., outer diameter, OD, and the inner diameter, ID surfaces). By ensuring one surface is held at a substantially lower temperature (e.g., ambient temperature) than the other surface (e.g., a temperature greater than one-half the melting temperature) a temperature gradient is developed.

So long as one surface is substantially below the recrystallization temperature (i.e., the temperature at which stress and strain-free grains nucleate at the expense of highly stressed ancient grains) of the chemically homogeneous material system, that surface will not undergo grain nucleation and growth processes. Conversely, the opposite surface will under recovery, recrystallization and subsequent grain growth processes as a result of thermally activated thermodynamic processes common to most material systems.

The resultant microstructure across the cross sectional dimension exhibits a transition in average linear grain size. The surface held to the lower temperature retains the initial ultra-fine grained, equiaxed microstructure. The opposing surface is characterized by a recrystallized, coarser grained microstructure (greater than 25 µm (25 microns), average linear grain size). The two surfaces will be connected by a transition region whereby the grain size increases fine to coarse grain. The resulting cross section, from one surface to another, thus can be characterized as a functionally graded microstructure.

By creating a functionally graded microstructure, the resultant strength and toughness attributes of both surfaces is substantially altered from the conventional monolithic form. Using the conventionally accepted Hall-Petch relationship that strength is inversely proportional to the square of the grain size, the strength increases as the grain size decreases.

The negative effect of the ultra-fine grained microstructure is a significant decrease in ductility (i.e., elongation of failure) and a corresponding reduction in toughness. By engineering the structure to place the higher stress microstructure at the site of greatest tensile loading (i.e., stress), nucleation of new defects or flaws is impeded without overall loss of ductility and toughness throughout the cross section.

Under stress controlled deformation conditions, the structure can be engineered to resist fatigue crack nucleation and subsequent propagation while minimizing structural cross sectional dimension (e.g., for tubular structure this dimension is the wall thickness). Fatigue prone structures are also microstructurally reengineered without change in the material chemical composition by the present invention.

In general terms, the present invention provides a method of fabricating an organized microstructure within an implantable medical device that comprises:
providing a monolithically homogeneous implantable biocompatible material comprising at least two exposed surfaces, said biocompatible material comprising an ultra-fine grained, equiaxed microstructure having an average linear grain size of less than 5 µm (5 microns); and
heating said biocompatible material under conditions in which one of said exposed surfaces is held at a first temperature T₁ and another of said exposed surfaces is held at a second temperature T₂, wherein T₁ is less than T₂ and T₁ is below a recrystallization temperature of the biocompatible material to provide an organized microstructure comprising a first surface region having said ultra-fine grained, equiaxed microstructure and a second surface region comprising a recrystallized, coarser grained microstructure having an average linear grain size of greater than 25 µm (25 microns), wherein said first and second surface regions are separated by a transition region.

In addition to the method described above, the present invention also relates to an implantable medical device that includes an organized microstructure which is formed by the method of the present invention. In general terms, the inventive implantable medical device comprises:
a biocompatible material comprising a first surface region having an ultra-fine grained, equiaxed microstructure having an average linear grain size of less than 5 µm (5 microns) and a second surface region comprising a recrystallized, coarser grained microstructure having an average linear grain size of greater than 25 µm (25 microns), wherein said first and second surface regions are separated by a transition region.

Embodiments of the invention will now be received by way of example with reference to the accompanying drawings, in which:
FIG. 1 is a pictorial representation (through a cross sectional view) depicting the inventive organized microstructure in a linear form that is produced utilizing the method of the present invention.
FIG. 2 is a pictorial representation (through a cross sectional view) depicting the inventive organized microstructure in a tubular form that is produced utilizing the method of the present invention.
FIG. 3 is a pictorial representation (through a top down perspective view) illustrating an embodiment of the present invention for forming an implantable medical device which includes the inventive organized microstructure in tubular form.

The present invention, which provides a method to fabricate an organized microstructure within an implantable medical device and an implantable medical device that includes the same, will now be described in greater detail by way of example by referring to the following discussion and drawings that accompany the present application. It is noted that the drawings are provided for illustrated purposes and, as such, they are not drawn to scale.

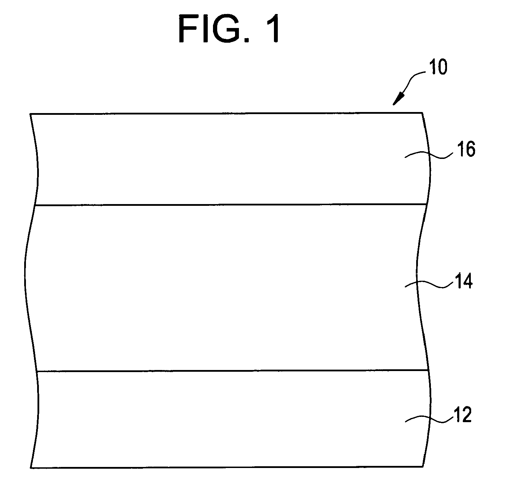

Reference is first made to FIG. 1 which is an enlarged cross sectional view depicting the inventive organized microstructure 10 that is produced utilizing the method of the present invention. As shown, the organized microstructure 10 includes a first surface region 12 having an ultra-fine grained, equiaxed microstructure (to be described in more detail herein below) and a second surface region 16 (to be described in more detail herein below) that has a recrystallized, coarser grained microstructure. The inventive microstructure 10 also includes a transition region 14 (to be described in more detail herein below) that separates the first surface region 12 from the second surface region 16.

In accordance with the present invention, the organized microstructure 10 shown in FIG. 1 is located within an implantable medical device. The term "implantable medical device" is used herein to denote a physical article used in medical treatment that can be introduced into living tissue. Suitable implantable medical devices that can include the inventive organized microstructure 10 include, but are not limited to: stents, blood filters, artificial heart valves, aneurismal coils, prosthetic grafts including stent-grafts, cochlear implants, visual prostheses, neurostimulators, muscular stimulators, deep brain stimulators and the like. The inventive organized microstructure 10 is comprised of an implantable biocompatible material. By "implantable" it is meant that the material can be inserted into a living site for medical usage. The term "biocompatible" denotes that the implantable material is compatible with a living tissue or a living organism by not being toxic or injurious and by not causing immunological reaction. Suitable implantable biocompatible materials that can be used in the present invention include, but are not limited to: metals, metal alloys, ceramic materials and polymers. Within the present invention, metal alloys are particularly preferred.

Suitable biocompatible materials such as elemental pure metals, metallic alloys, ceramic materials, and/or polymeric materials may be considered in this embodiment. It is important to note that any number of alloys and engineered (i.e., compositionally pure monolithic) metals, including iron-based alloys, cobalt-based alloys, refractory-based alloys, refractory metals, precious metal-based alloys, precious metals and titanium-based alloys may be used in accordance with the present invention.

In yet another exemplary embodiment, an intraluminal scaffold element may be fabricated from a non-metallic material such as a polymeric material including non-crosslinked thermoplastics, cross-linked thermosets, composites and blends thereof. There are typically three different forms in which a polymer may display the mechanical properties associated with solids; namely, as a crystalline structure, as a semi-crystalline structure and/or as an amorphous structure. All polymers are not able to fully crystallize, as a high degree of molecular regularity within the polymer chains is essential for crystallization to occur. Even in polymers that do substantially crystallize, the degree of crystallinity is generally less than 100 percent. Within the continuum between fully crystalline and amorphous structures, there are two thermal transitions possible; namely, the crystal-liquid transition (i.e., melting point temperature, Tm) and the glass-liquid transition (i.e., glass transition temperature, Tg). In the temperature range between these two transitions there may be a mixture of orderly arranged crystals and chaotic amorphous polymer domains.

The polymers, which term denotes a chemical compound with a high molecular weight consisting of a number of structural units linked together, that can be used in the present invention include, but are not limited to: silicone polymers (i.e., organosiloxane), polyurethanes, polyamides, parylene, fluoropolymers such as, for example, Teflon, polyolefins such as, for example, polyethylene and polypropylene, collagen, chitin, alginate, polyvinyl pyrrolidone, polyethylene glycol, polyethylene oxide, polyvinyl alcohol, polyglycoal lactic acid, polylactic acid, polycaprolactone, polyamino acids and hydrogels such as, for example, carboxymethyl cellulose.

As indicated above, the organized microstructure 10 includes a first surface region 12 having an ultra-fine grained, equiaxed microstructure. The term "equiaxed microstructure" denotes a structure in which the crystalline granularity is substantially equal in spatial dimension, when assessed on a continuum of cross-sectional orientations, and has measurable mechanical attributes that are homogenous and isotropic as a result of the intended microstructure. The term "ultra-fine grained" denotes an average linear grain size of less than about 5 µm (5 microns). More typically, the ultra-fined grained, equiaxed microstructure has an average linear grain size of about 1 µm (1 micron) to about 100 nanometers. The average linear grain size is determined by industrially accepted consensus standards for determining and analyzing microstructural cross-sections as is well known in the art.

The first surface region 12 is characterized as having a relatively low ductility (i.e., elongation to failure) on the order of about 10% (engineering strain) or less and a relatively low toughness. The first surface region 12 is thus characterized as being of a higher mechanical strength than the second surface region 16. It is noted that the first surface region 12 is engineered in the present invention to be located at a positioned within the implantable medical device in which stress caused by tensile loading is the highest.

The thickness of the first surface region 12 is dependent about the size of the implantable medical device. For most implantable medical devices, the thickness of the first surface region 12 is typically from about 100 nanometers to about 5 µm (5 microns), with a thickness from about 200 nanometers to about 2 µm (2 microns) being even more typical.

The transition region 14 which is located between the first surface region 12 and the second surface region 16 is characterized as an area within the inventive organized microstructure 10 in which there is a change from an ultra-fine to coarse grained microstructure. Because of the presence of the transition region 14 between the first surface region 12 and the second surface region 16, the inventive organized microstructure 10 can be characterized as a functionally graded microstructure. Within the transition region 14, the average linear grain size increases from ultra-fine (located at or near the first surface region 12) to coarse (located at or near the second surface region 14). The thickness of the transition region 14 may also vary depending on the size of the implantable medical device. Typically, and for most of the conventional implantable medical devices, the thickness of the transition region 14 is from about 100 nanometers to about 30 µm (30 microns), with a thickness from about to 2 µm (2 microns) about 20 µm (20 microns) being even more typical.

The inventive structure shown in FIG. 1 also includes a second surface region 16. The second surface region 16 comprises a recrystallized, coarser grained, equiaxed microstructure having an average linear grain size of greater than 25 µm (25 microns). More typically, the average linear grain size of the recrystallized microstructure is from about 40 to about 50 µm (about 40 to about 50 microns) for an intravascular coronary stent. It is important to note however that the average linear grain size will vary depending upon the application and intended device.

The second surface region 16 is characterized as having a relatively high ductility (i.e., elongation to failure) on the order of about 40% (engineering strain) or greater and a relatively high toughness. The second surface region 16 is thus characterized as being of a lower mechanical strength than the first surface region 12. It is noted that the second surface region 16 is engineered in the present invention to be located at a position within the implantable medical device in which stress caused by tensile loading is the lowest. By engineering the device to place the higher mechanical strength microstructure (i.e., first surface region 12) at the site of greatest tensile loading (i.e., stress), nucleation of new defects or flaws will be impeded without the overall loss of ductility and toughness.

The thickness of the second surface region 16 is dependent about the size of the implantable medical device. For most implantable medical devices, the thickness of the second surface region 16 is typically from about 70 µm (70 microns) to about 90 µm (90 microns) or greater, with a thickness from about 75 to about 85 µm (about 75 to about 85 microns) being even more typical.

It is noted that under stress controlled deformation conditions, medical implantable devices including the inventive organized microstructure can be engineered to resist fatigue crack nucleation and subsequent propagation, while minimizing structural cross sectional dimension. By "stress controlled deformation conditions" it is meant that the induced loads and resulting deformation is regulated by the *in vivo* environment.

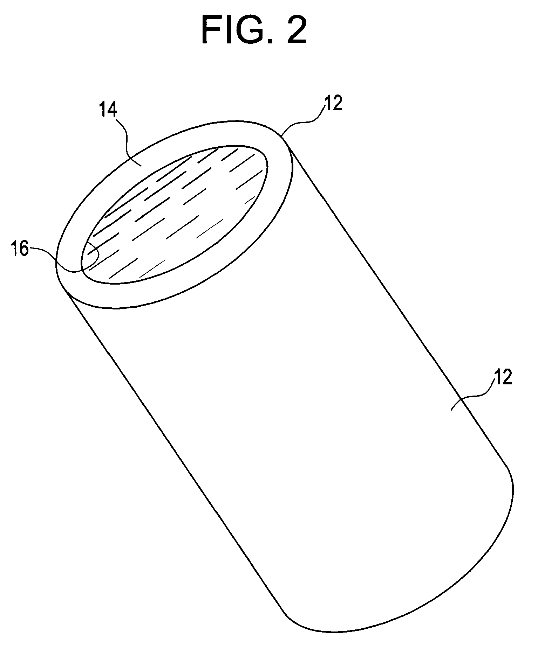

FIG. 2 is an enlarged cross sectional view depicting the inventive organized microstructure 10 in a tubular form. In the specific embodiment shown, the outer diameter of the tube is comprised of the first surface region 12 and the inner diameter of the tube is comprised of the second surface region 16. It is also possible to construct a tubular form in which these surfaces are reversed.

The structure shown in FIGS. 1 and 2, or any other equivalent structure, is formed by first providing a monolithically homogeneous implantable biocompatible material that includes at least two exposed surfaces. In accordance with the present invention, the initial biocompatible material has an ultra-fine grained, equiaxed microstructure having an average linear grain size of less than 5 µm (5 microns), with an average linear grain size from about 200 nanometers to about 2 µm (2 microns) being more highly preferred.

The monolithically homogeneous implantable biocompatible material is typically formed utilizing conventional processes well known in the art. In this exemplary embodiment, the initial raw material product form that is required to initiate the thermomechanical processing that will ultimately yield a desired small diameter, thin-walled tube, appropriate for interventional devices, is a modestly sized round bar (e.g., 25.4 mm (one inch) in diameter round bar stock) of predetermined length. In order to facilitate the reduction of the initial bar stock into a much smaller tubing configuration, an initial clearance hole must be placed into the bar stock that runs the length of the product. These tube hollows (i.e., heavy walled tubes) may be created by 'gun-drilling' (i.e., high depth to diameter ratio drilling) the bar stock. Other industrially relevant methods of creating the tube hollows from round bar stock may be utilized by those skilled-in-the-art of tube making.

Consecutive mechanical cold-finishing operations such as drawing through a compressive outer-diameter (OD), precision shaped (i.e., cut), circumferentially complete, diamond die using any of the following internally supported (i.e., inner diameter, ID) methods, but not necessarily limited to these conventional forming methods, such as hard mandrel (i.e., relatively long traveling ID mandrel also referred to as rod draw), floating-plug (i.e., relatively short ID mandrel that 'floats' within the region of the OD compressive die and fixed-plug (i.e., the ID mandrel is 'fixed' to the drawing apparatus where relatively short work pieces are processed) drawing. These process steps are intended to reduce the outer-diameter (OD) and the corresponding wall thickness of the initial tube hollow to the desired dimensions of the finished product.

When necessary, tube sinking (i.e., OD reduction of the workpiece without inducing substantial tube wall reduction) is accomplished by drawing the workpiece through a compressive die without internal support (i.e., no ID mandrel). Conventionally, tube sinking is typically utilized as a final or near-final mechanical processing step to achieve the desired dimensional attributes of the finished product. The initial monolithic material may be a coating that is applied to an implantable medical device or it can comprise the entire implantable medical device itself.

Although not practically significant, if the particular compositional formulation will support a single reduction from the initial raw material configuration to the desired dimensions of the finished product, in process heat-treatments will not be necessary. Where necessary to achieve intended mechanical properties of the finished product, a final heat-treating step is utilized.

Conventionally, all metallic alloys in accordance with the present invention will require incremental dimensional reductions from the initial raw material configuration to reach the desired dimensions of the finished product. This processing constraint is due to the material's ability to support a finite degree of induced mechanical damage per processing step without structural failure (e.g., strain-induced fracture, fissures, extensive void formation, etc.).

In order to compensate for induced unintended mechanical damage (i.e., cold-working) during any of the aforementioned cold-finishing steps, periodic thermal heat-treatments are utilized to stress-relieve, (i.e., minimization of deleterious internal residual stresses that are the result of processes such as cold-working) thereby increasing the workability (i.e., ability to support additional mechanical damage without measurable failure) of the workpiece prior to subsequent reductions. These thermal treatments are typically, but not necessarily limited to, conducted within a relatively inert environment such as an inert gas furnace (e.g., nitrogen, argon, etc.), an oxygen rarified hydrogen furnace, a conventional vacuum furnace and under less common process conditions, atmospheric air. When vacuum furnaces are utilized, the level of vacuum (i.e., sub atmospheric pressure), typically measured in units of mm Hg or torr (where 1 mm Hg is equal to 1 unit torr), shall be sufficient to ensure that excessive and deteriorative high temperature oxidative processes are not functionally operative during heat treatment. This process may usually be achieved under vacuum conditions of 10⁻⁴ mm Hg (0.0001 torr) or less (i.e., lower magnitude).

The final cold-finishing steps will be determined by one skilled-in-the-art of process metallurgy to ensure that the as-processed microstructure can be characterized to be comprised of an organized microstructure having an ultra-fine grained, equiaxed microstructure. The term "equiaxed microstructure" denotes a structure in which the crystalline granularity is substantially equal in spatial dimension, when assessed on a continuum of cross-sectional orientations, and has measurable mechanical attributes that are homogenous and isotropic as a result of the intended microstructure. The term "ultra-fine grained" denotes an average linear grain size of less than about 5 µm (5 microns). More typically, the ultra-fined grained, equiaxed microstructure has an average linear grain size of about 1 µm (1 micron) to about 100 nanometers. The average linear grain size is determined by industrially accepted consensus standards for determining and analyzing microstructural cross-sections as is well known in the art.

The final thermomechanical processing steps used to ensure that an ultra-fine grained, equiaxed microstructure in the as-processed material that is characterized by the exemplary embodiment described herein shall comprise additional high temperature heat treating steps, as needed, to ensure that the yielded raw material is sufficiently ductile to facilitate necessary handling and process steps required to manufacture intended finished product forms and structures without unintended raw material failures. These unintended raw material failures are typically characterized by deleterious brittle fracture. The time, temperature, heat treating environment, cooling medium and corresponding rate of cooling after sufficient high temperature exposure as well as the specific processing schedule are all process factors that are readily determinable as a function of the material system processed and are typically know by those skilled in-the-art of process metallurgy.

The biocompatible material is then heated under conditions in which one of the exposed surfaces is held at a first temperature T₁ and another of the exposed surfaces is held at a second temperature T₂, wherein T₁ is less than T₂ and T₁ is below a recrystallization temperature of the biocompatible material. Under these conditions, the inventive organized microstructure shown in FIGS. 1 and 2, for example, is provided.

In accordance with the present invention, the surface that is held at T₁ will maintain the ultra-fine microstructure of the initial monolithic biocompatible material, while the other surface that is held at T₂ recrystallizes into the coarser grained microstructure described above. The surface that is held at T₂ goes through two stages of microstructural evolution as a result of the imparted surface specific thermal energy. Upon heating above the recrystallization temperature of the material, the highly stressed, ancient crystallographic grains that were retained from the primary melt solidification during ingot stage processing will exhibit a form of internal stress relief by nucleation of stress and strain free grains along internal defect laden zones (i.e., crystallographic planes and corresponding 'slip' or shear directions within the principal crystallographic planes that characterize each distinct grain). The recrystallization temperature denotes the temperature where nucleation of secondary crystallographic stress and strain free grains are thermodynamically stable and as such are energetically favorable to exist as opposed to the primal grains. As these newly nucleated grains grow at the energetic expense of the parent grains, the principal nucleation mechanism will give way to the rate-controlling grain-growth aspects that will be expected to dominate the overall kinetics of the surface oriented microstuctural ripening or change that is characteristic of the exemplary embodiment presented herein.

The heating step may be performed utilizing a cold source (e.g., a liquid nitrogen cold finger) to hold the temperature at T₁. Typically, temperature T₁ is at nominal room temperature (20°-40°C) or at a temperature that is below nominal room temperature, e.g., T₁ may be at the temperature of liquid nitrogen, -194°C (at standard atmospheric pressure). In one embodiment, a localized heat sink is used to heat one of the surfaces to T₂. It is noted that temperature T₂ is typically greater than about ½ the melting temperature of a selected biocompatible material. In addition as previously discussed, T₂ will also be greater than the recrystallization temperature of the selected biocompatible temperature. For instance, surface specific processing at the higher temperature (i.e., T₂) often requires high temperature treatment (typically on the order of about 0.75 to 0.90 homologous temperature or greater, where the homologous temperature is the industrially accepted liquidus temperature also generally referred to as the melting temperature of the material) of the metallic device to recrystallize its microstructure to reduce grain size and retained residual stress in the completed product form.

In addition to a heat sink, direct contact conduction, infrared heating, laser heating delivered by thermal energy, radio frequency (RF) induction heating through eddy current inducement by direct coupling of the RF coil to the workpiece or by RF coupling to a conductive susceptor or adjacent heating element which will heat the corresponding workpiece by secondary conductive, convective and/or radiative heating, can be employed.

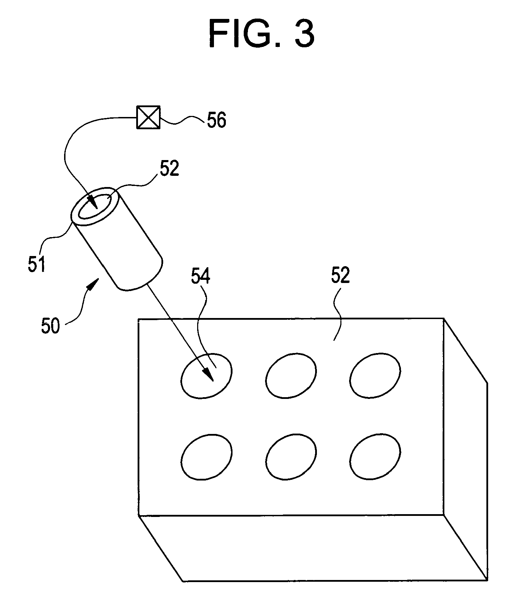

FIG. 3 shows a pictorial representation of a tubular structure comprising the monolithically homogeneous implantable biocompatible material 50 having surfaces S 1 and S2. S2 is heated to a temperature T₂ utilizing a thermal heat source 56, while S1 is held at temperature T₁ utilizing a cold sink 52 that includes opening 54 in which the biocompatible material 50 can be positioned within.

It is noted that the present invention provides a method in which a functionally organized microstructure can be formed within an implantable medical device in which crack nucleation and propagation processes are impeded. Moreover, the present invention also for: (i) fatigue prone structures to be reengineered at the microstructural level; (ii) the highest strength regions of the microstructure can be placed where the highest levels of stress are expected as a result of prior structural analysis; (iii) as a result of prior characteristics, the microstructure can be designed with less load carrying cross sectional area (i.e., in the case of a tubular structure, a thinner wall dimension); and (iv) engineered strength attributes both through the cross sectional dimension of the microstructure (i.e., in the case of a tubular structure, the wall dimension and longitudinal axis, respectively.

## Claims

1. A method comprising:
providing a monolithically homogeneous implantable biocompatible material comprising at least two exposed surfaces, said biocompatible material comprising an ultra-fine grained, equiaxed microstructure having an average linear grain size of less than 5 µm (5 microns); and
heating said biocompatible material under conditions in which one of said exposed surfaces is held at a first temperature T₁ and another of said exposed surfaces is held at a second temperature T_{2.} wherein T₁ is less than T₂ and T₁ is below a recrystallization temperature of the biocompatible material to provide an organized microstructure comprising a first surface region having said ultra-fine grained, equiaxed microstructure and a second surface region comprising a recrystallized, coarser grained microstructure having an average linear grain size of greater than 25 µm (25 microns), wherein said first and second surface regions are separated by a transition region.

2. The method of Claim 1 wherein said biocompatible material comprises a metal, a metal alloy, a ceramic material or a polymer.

3. The method of Claim 2 wherein said biocompatible material is a metal alloy or a compositionally pure monolithic metal that is selected from the group consisting of iron-based alloys, cobalt-based alloys, refractory metal-based alloys, precious metal-based alloys, precious metals and titanium-based alloys.

4. The method of Claim 1. wherein said biocompatible material is process to be in tubular form.

5. The method of Claim 1 wherein the average linear grain size of said first surface region having said ultra-fine grained, equiaxed microstructure is from about 1 µm (1 micron) to about 100 nanometers.

6. The method of Claim 1 wherein said average linear grain size of said recrystallized, coarser grained microstructure is from about 40 µm (40 microns) to about 50 µm (50 microns).

7. The method of Claim 1 wherein T₁ is at nominal room temperature or a temperature that is below nominal room temperature.

8. The method of Claim 7 wherein T₁ is at the temperature of liquid nitrogen at standard atmospheric conditions.

9. The method of Claim 1 wherein T₂ is at a temperature that is greater than about ½ the melting point of said biocompatible material.

10. The method of Claim 9 wherein T₂ is from about 0.75 to about 0.90 homologous temperature or greater, where the homologous temperature is the industrially accepted liquidus temperature.

11. An implantable medical device comprising:
a biocompatible material comprising a first surface region having an ultra-fine grained, equiaxed microstructure having an average linear grain size less than 5 µm (5 microns) and a second surface region comprising a recrystallized, coarser grained microstructure having an average linear grain size of greater than 25 µm (25 microns), wherein said first region and second surface regions are separated by a transition region.

12. The implantable medical device of Claim 11 wherein said biocompatible material comprises a metal, a metal alloy, a ceramic material or a polymer.

13. The implantable medical device of Claim 12 wherein said biocompatible material is a metal alloy or a compositionally pure monolithic metal that is selected from the group consisting of iron-based alloys, cobalt-based alloys, refractory metal-based alloys, precious metal-based alloys, precious metals and titanium-based alloys.

14. The implantable medical device of Claim 11 wherein said biocompatible material is process to be in tubular form.

15. The implantable medical device of Claim 11 wherein the average linear grain size of said first surface region having said ultra-fine grained, equiaxed microstructure is from about 1 µm (1 micron) to about 100 nanometers.

16. The implantable medical device of Claim 11 wherein said average linear grain size of said recrystallized, coarser grained microstructure is from greater than about 40 µm (40 microns) to about 50 µm (50 microns).

17. The implantable medical device of Claim 11 wherein said implantable medical device is one of a stent, a blood filter, an artificial heart valve, vascular occluders, aneurismal coils, prosthetic grafts including stent-grafts, a cochlear implant, a visual prosthesis, a neurostimulator, a muscular stimulator or a deep brain stimulator.

18. The implantable medical device of Claim 11 wherein said first surface region has a ductility from about 10% (engineering strain to failure) or less.

19. The implantable medical device of Claim 11 wherein said second surface region has a ductility from about 40% (engineering strain to failure) or greater.

20. The implantable medical device of Claim 11 wherein said transition region is an area in which there is a change from said ultra-fine microstructure at a surface nearest to the first surface region to said coarser grained microstructure at a surface nearest to the second surface region.
